# EUROPÄISCHE PATENTSCHRIFT

(11) **EP 2 382 323 B1**
(45) Veröffentlichungstag und Bekanntmachung des Hinweises auf die Patenterteilung: **29.07.2015**
(21) Anmeldenummer: 09798936.2
(22) Anmeldetag: 23.12.2009
(51) Int. Cl.: C12N 15/10, C12Q 1/68

(54) **PRÄPARATION UND AMPLIFIKATION VON NUKLEINSÄUREN MITTELS MAGNETISCHER PARTIKEL**
PREPARATION AND AMPLIFICATION OF NUCLEIC ACIDS BY MEANS OF MAGNETIC PARTICLES
PRÉPARATION ET AMPLIFICATION D'ACIDES NUCLÉIQUE PAR DES PARTICULES MAGNÉTIQUES

(30) Priorität: 23.12.2008 DE 102008055120
(43) Veröffentlichungstag der Anmeldung: 02.11.2011
(73) Patentinhaber: Qiagen GmbH, 40724 Hilden (DE)
(72) Erfinder: HIMMELREICH, Ralf, 40764 Langenfeld (DE); ROTHMANN, Thomas, 40764 Langenfeld (DE); FABIS, Roland, 51375 Leverkusen (DE); ERBACHER, Christoph, 42781 Haan (DE)
(74) Vertreter: Roth, Carla
(86) Internationale Anmeldenummer: PCT/EP2009/067882
(87) Internationale Veröffentlichungsnummer: WO 2010/072822

(56) Entgegenhaltungen:
- EP-A1- 1 371 736
- WO-A1-95/13368
- WO-A1-2008/147382
- DE-A1-102005 049 976
- VOLKHEIMER ALICIA D ET AL: "A single cell analysis of immunoglobulin genes in chronic lymphocytic leukemia (CLL): Progressive somatic mutations in the immunoglobulin heavy and light chains contribute to intraclonal diversification in CLL" BLOOD, AMERICAN SOCIETY OF HEMATOLOGY, US, Bd. 102, Nr. 11, 9. Dezember 2003 (2003-12-09), Seite 666A, XP009137342 ISSN: 0006-4971
- KAUPPINEN J ET AL: "Mycobacterium malmoense-specific nested PCR based on a conserved sequence detected in random amplified polymorphic DNA fingerprints." JOURNAL OF CLINICAL MICROBIOLOGY MAY 1999 LNKD- PUBMED:10203504, Bd. 37, Nr. 5, Mai 1999 (1999-05), Seiten 1454-1458, XP002595592 ISSN: 0095-1137
- DATABASE WPI Week 200313 Thomson Scientific, London, GB; AN 2003-136576 XP002595629 & KR 2002 029 476 A (PARK J C) 19. April 2002 (2002-04-19)
- KIM DUWOON ET AL: "Detection of hepatitis a virus from oyster by nested PCR using efficient extraction and concentration method." JOURNAL OF MICROBIOLOGY (SEOUL, KOREA) AUG 2008 LNKD- PUBMED:18758735, Bd. 46, Nr. 4, August 2008 (2008-08), Seiten 436-440, XP002595525 ISSN: 1225-8873
- LU YANHUI ET AL: "Use of whole genome amplification to rescue DNA from plasma samples", BIOTECHNIQUES, INFORMA HEALTHCARE, US, vol. 39, no. 4, 1 October 2005 (2005-10-01), pages 511-515, XP001538765, ISSN: 0736-6205, DOI: 10.2144/000112005

## Beschreibung

Die Erfindung betrifft eine Vorbereitung einer biologischen Probe für die Durchführung von Nachweisen und Untersuchungen.

Aus dem Stand der Technik ist bekannt, für die Vorbereitung einer biologischen Probe zunächst den Inhalt einer biologischen Probe zugänglich zu machen (bekannt unter der Bezeichnung "Lyse" oder "Aufschließen"), Bestandteile des freigesetzten Inhalts der biologischen Probe an oder auf einem festen Stütz- oder Trägermaterial selektiv zu binden (bekannt unter der Bezeichnung "Binden"), unerwünschte Bestandteile vom festen Stütz- oder Trägermaterial zu beseitigen (bekannt unter der Bezeichnung "Waschen") und die gewünschten Bestandteile, nämlich Nukleinsäuren anschließend vom festen Stütz- oder Trägermaterial zu lösen (bekannt unter der Bezeichnung "Elution"). Zumindest ein gesuchter Bestandteil, also ein definierter Teil eines DNA- oder RNA-Strangs wird schließlich beispielsweise mittels einer Polymerase-Kettenreaktion (PCR) vervielfältigt, also amplifiziert.

Der PCR-Prozess besteht aus einer Anzahl von beispielsweise 25-50 Zyklen, die in einem Thermocycler durchgeführt werden. Die folgenden Angaben stellen Richtwerte dar. Meist muss eine PCR auf die spezifische Reaktion hin optimiert werden.

Jeder Zyklus besteht aus drei Schritten:
1. Denaturierung (Melting, Schmelzen): Zunächst wird die doppelsträngige DNA auf 94-96°C erhitzt, um die Stränge zu trennen. Die Wasserstoffbrückenbindungen, die die beiden DNA-Stränge zusammenhalten, werden aufgebrochen. Im ersten Zyklus wird die DNA oft für längere Zeit erhitzt (Initialisierung), um sicherzustellen, dass sich sowohl die Ausgangs-DNA als auch die Primer vollständig voneinander getrennt haben und nur noch Einzelstränge vorliegen. Manche (so genannte hot start-) Polymerasen müssen durch eine noch längere anfängliche Erhitzungs-Phase (bis zu 15 Minuten) aktiviert werden.
2. Primerhybridisierung (primer annealing): Die Temperatur wird ca. 30 Sekunden lang auf einer Temperatur gehalten, die eine spezifische Anlagerung der Primer an die DNA erlaubt. Die genaue Temperatur wird hierbei durch die Länge und die Sequenz der Primer bestimmt (bzw. der passenden Nukleotide im Primer, wenn durch diesen Mutationen eingeführt werden sollen = sitedirected Mutagenesis). Wird die Temperatur zu niedrig gewählt, können sich die Primer unter Umständen auch an nicht-100-%-komplementären Sequenzen anlagern und so zu unspezifischen Produkten ("Geisterbanden") führen. Wird die Temperatur zu hoch gewählt, ist die thermische Bewegung der Primer u. U. so groß, dass sie sich nicht richtig anheften können, so dass es zu gar keiner oder nur ineffizienter Produktbildung kommt. Die Temperatur, welche die beiden oben genannten Effekte weitgehend ausschließt, liegt normalerweise 2-3°C unter dem Schmelzpunkt der Primersequenzen; dies entspricht meist einer Temperatur von 55-65°C.
3. Elongation (Polymerisation, Verlängerung, Amplifikation): Schließlich füllt die DNA-Polymerase die fehlenden Stränge mit freien Nukleotiden auf. Sie beginnt am 3'-Ende des angelagerten Primers und folgt dann dem DNA-Strang. Der Primer wird nicht wieder abgelöst, er bildet den Anfang des neuen Einzelstrangs. Die Temperatur hängt vom Arbeitsoptimum der verwendeten DNA-Polymerase ab (68-72°C). Dieser Schritt dauert etwa 30 Sekunden je 500 Basenpaare, variiert aber in Abhängigkeit von der verwendeten DNA-Polymerase. Übliche Thermocycler kühlen die Reaktionsansätze nach Vollendung aller Zyklen auf 4-8 °C herunter, so dass eine PCR am Abend angesetzt werden kann und die Proben am Morgen darauf weiter verarbeitet werden können.

Im ersten Zyklus entstehen zunächst DNA-Einzelstränge, welche in 5'-Richtung länger als die Zielsequenz sind. Dies ist damit zu erklären, das lediglich ein Startpunkt (Primer) nicht aber ein Endpunkt exakt festgelegt ist. Der Abbruch der Strangsynthese erfolgt dabei spätestens durch die Strangtrennung im folgenden Denaturierungsschritt. Im zweiten Zyklus stehen die eingesetzte DNA sowie die gerade gebildeten DNA-Stränge zur Verfügung. An Ersterer erfolgt derselbe Prozess wie im ersten Zyklus. An die neu gebildeten DNA-Einzelstränge, welche 3' bereits dort Enden wo sie sollen, lagern sich nun Primer in der 5'-Region an. Die nun gebildeten Stränge haben keinen 5'-Überhang, da die Polymerase auf dem Template in Richtung des 3'-Endes liest. Am Ende des zweiten Zyklus stehen damit erstmals Produkte der gewünschten Länge. In den folgenden Zyklen vermehren sich die gewünschten Produkte exponentiell (da sie selbst als Matrize für weitere Strangsynthesen dienen), während die ungewünschten langen Produkte (siehe Produkte des ersten Zyklus) nur linear ansteigen (nur eingesetzte DNA dient als Matrize). Dies ist der theoretische Idealfall, in der Praxis fallen zudem in geringem Maße auch kürzere Fragmente als die gewünschte Ziel-DNA an. Diese kurzen Fragmente häufen sich vor allem in den späten Zyklen an, wodurch meist nur etwa 30 Zyklen durchlaufen werden, um insgesamt vorwiegend DNA der gewünschten Länge und Sequenz zu erhalten.

Eine so vorbereitete Probe wird im Anschluss daran beispielsweise mittels einer Agarose-Gelelektrophorese identifiziert. Ein Gel wird beispielsweise durch Aufkochen von Agarose in einem Puffer, beispielsweise TBE-Puffer, hergestellt. Lange Fäden aus Agarosepolymeren werden so zu einem Gel vernetzt. In der Regel werden den Gelen bereits bei der Herstellung Hilfsstoffe zur Sichtbarmachung der aufgetrennten Moleküle zugesetzt. Im Falle von DNA handelt es sich dabei meist um Ethidiumbromid. Mittels einer Agarose-Gelelektrophorese werden Nukleinsäure-Stränge (RNA oder DNA) nach ihrer Größe getrennt, und ihre Größe werden durch Vergleich mit Strängen bekannter Größe bestimmt. Je höher die Agarose konzentriert ist, desto kleiner sind die Poren, die sich in dem Gel befinden. Die Gelelektrophorese funktioniert wie ein Sieb für Moleküle. Ein elektrisches Feld wird verwendet, um die negativ geladenen Nukleinsäure-Moleküle durch die Gelmatrix zu ziehen, wobei die kleineren Moleküle sich schneller durch das Gel bewegen können und somit eine Auftrennung der Stränge nach ihrer Größe ermöglicht wird. Aufgrund von zugesetzten Hilfsstoffen können die so aufgetrennten Nukleinsäuren in der Regel mit UV-Licht sichtbar gemacht werden. Es kann so ermittelt werden, ob ein gesuchter Teil eines DNA-Strangs durch die PCR vervielfältigt wurde und folglich die Probe ursprünglich die gesuchten DNA - Stränge aufwies. Derartige Nachweise werden u. a. bei Vaterschaftstests sowie in der Forensik geführt.

Beispielsweise auf dem Gebiet der Forensik steht in der Regel nur eine begrenzte, geringe Menge an Probenmaterial zur Verfügung. In solchen Fällen ist es wichtig, eine hohe PCR-Sensitivität zu erhalten. Die PCR-Sensitivität ist ein Maß für die Vervielfältigung des gesuchten Teils eines DNA Strangs. Je sensitiver die PCR ist, umso besser und schneller wird der entsprechende DNA Strang vervielfältigt.

Ein automatisiertes Verfahren für die Vorbereitung einer biologischen Probe, welches die Schritte Lyse, Binden, Waschen, Elution und PCR umfasst, ist der europäischen Patentanmeldung EP 2087934 zu entnehmen: Eine biologische Probe wird in einen Behälter gebracht, der einen beispielsweise aus einem SilicaGel bestehenden Filter aufweist. Unterhalb des Filters ist ein Zu- bzw. Abfluss für Flüssigkeit vorgesehen. Zunächst wird eine biologische Probe insbesondere in diesem Behälter mit einem Lysepuffer aufgeschlossen. Nach dem Aufschließen der Probe wird der Lysepuffer durch den Filter hindurch abgesaugt. Es wird dadurch zugleich erreicht, dass die durch Aufschluss freigesetzte Nukleinsäure an den Filter bindet. Das Binden der Nukleinsäure an den Filter wird durch eine anschließende Zugabe von Ethanol in das Gefäß verstärkt. Mit Hilfe von Waschpuffern wird danach gewaschen, um so Fette, Lipide und Proteine zu entfernen. Nach dem Waschen wird die gebundene Nukleinsäure mit Hilfe von Elutionsflüssigkeit von dem Filter gelöst. Die Elutionsflüssigkeit wird zusammen mit den nun darin befindlichen Nukleinsäuren einer PCR-Kammer zugeführt, um darin die gewünschte Polymerase-Kettenreaktion durchzuführen.

Aus der europäischen Patentanmeldung EP 2087934 geht weiter hervor, dass Ergebnis der PCR mit Hilfe eines Agarosegels elektrophoretisch aufzutrennen und die aufgetrennten Nukleinsäurefragmente in einem Ethidiumbromidhaltigem Wasserbad einzufärben. Durch Anregung mit UV-Licht werden die eingefärbten Nukleinsäurefragmente sichtbar und so nachgewiesen. Die Bindung erfolgt auf Basis von chaotrop Chemie. Es wird dann unter Hoch-Salz (also einer Lösung mit hoher Salzkonzentration) gebunden. Eine Ethanol - Elution erfolgt mit Niedrig-Salz, also einer Lösung mit niedriger Salzkonzentration. Wird dagegen ein Anionenaustauscher eingesetzt, so erfolgt die Bindung unter Niedrig-Salz und Elution unter Hoch-Salz.

Die beschriebene Vorbereitung einer biologischen Probe, welches die genannten Schritte Lyse, Binden, Waschen, Elution und PCR umfasst, wird Referenzstandard oder auch Goldstandard genannt. Mit dem Goldstandard kann eine hohe PCR-Sensitivität erreicht werden. Es genügt dann eine geringe Menge an Probenmaterial, um den gesuchten DNA - Strang hinreichend vervielfältigen zu können. In der Regel umfasst der Referenzstandard eine Standard Nukleinsäurepräparation (mit den Schritten Lyse, Binden, Waschen und Elution) und nachfolgend eine Standard-PCR und nicht die deutlich aufwändigere, aber auch deutlich sensitivere nested-PCR, da die Standard-PCR für die Durchführung der gewünschten Nachweise in der Regel genügt. PCRs sind prinzipiell anfällig gegenüber Kontaminationen durch Inhibitoren, die die Effizienz der Reaktion vermindern können. Der Vorteil bei einer nested-PCR ist, dass selbst wenn die erste PCR leichte Inhibition aufweist - so kann durch die zweite PCR, die dann in der Regel ohne Inhibitionsprobleme verläuft, trotz primärer Inhibition in der ersten PCR eine hohe Sensitivtät erzielt werden. Wenn dagegen eine "einzelne" PCR (Referenzstandard) zum Beispiel eine qPCR Kontaminationen durch Inhibitoren aufweist, so ist die Effizienz und somit auch die Sensitivtät der Reaktion vermindert. Problematisch ist allerdings bei einer nested-PCR im Vergleich zur Standard-PCR das Auftreten einer Kreuzkontamination. Durch Aerosole und andere "Unfälle" ist die Gefahr für falsch positive Ergebnisse im Fall der nested-PCR sehr hoch.

Eine Vorbereitung einer biologischen Probe nach dem Goldstandard ist weiter der Druckschrift WO 93/1 1221 zu entnehmen. Hieraus ist bekannt, biologische Proben durch Verwendung von Enzymen, wie zum Beispiel Proteinase K, Lysozym und Detergentien wie SDS, Brij, Triton-X-100, Tween 20, DOC und Chemikalien wie Natriumhydroxid, Guanidin - Hydrochlorid und Guanidin-Isothiocyanat aufzuschließen. Nach dem Aufschließen und Entfernen von unerwünschten Zelltrümmern wird die zu isolierende Nukleinsäure an einen Anionenaustauscher gebunden. Der Druckschrift ist weiter zu entnehmen, dass nach dem Binden zunächst durch Waschen abzutrennende Substanzen entfernt werden, um dann die gebundenen Nukleinsäuren mit einem Elutionspuffer hoher Ionenstärke wieder abzulösen. Diesem Stand der Technik ist weiter zu entnehmen, dass die weiteren Verfahrensoperationen nur mit Pufferbedingungen möglich sind, die geringere Ionenstärken aufweisen. Insbesondere vor der Durchführung einer PCR muss im Anschluss an die Elution zunächst die Nukleinsäure entsalzt werden, was nachteilhaft einen entsprechend vergrößerten Aufwand erfordert. Als Anionenaustauscher kann gemäß der Druckschrift WO 93/1 1221 ein handelsübliches Material ausgewählt werden, welches eine Bindung der zu isolierenden Nukleinsäure unter den jeweiligen Präparationsbedingungen erlaubt. Die aus der WO 93/11221 bekannten Anionenaustauscher sind vorzugsweise oberflächenmodifizierte Träger aus einer Matrix, vorzugsweise bestehend aus Agarose, Dextran, Zellulose, Acrylamid, Polyvinylalkohol, Polystyrol, Glas, Aluminiumoxid, Titandioxid, Zirkondioxid oder Silicagel, wie zum Beispiel DEAE-Sepharose^{R}, Q-Sepharose^{R}, DEAE-Sephadex^{R}, DEAE-Toyopearl^{R}, Amberlite^{R}, Nukleogen^{R}. Die Anionenaustauscher können poröse Trägermaterialien mit einer zur Wechselwirkung geeigneten inneren Oberfläche hoher Kapazität oder nicht poröse Trägermaterialien sein, die nur auf der äußeren Oberfläche eine Wechselwirkung mit dem zu trennenden Gemisch eingeht. Ganz besonders bevorzugt handelt es sich bei dem Anionenaustauscher um ein Material auf Basis von Silicagel, das eine Partikelgröße von 1 bis 250 µm, vorzugsweise 10 bis 50 µm und ganz besonders bevorzugt 15 bis 25 µm und einen Porendurchmesser von 1 bis 2.500 nm, bevorzugt 10 bis 500 nm, besonders bevorzugt 100 bis 400 nm aufweist. Als Anionenaustauschermaterial hat sich insbesondere ein Material mit hoher Oberflächenladung und hoher Bindungskapazität für Nukleinsäuren erwiesen.

Die Modifizierung des Silicagels erfolgt laut der WO 93/11221 vorzugsweise durch Silanisierung des Trägermaterials, wie beispielsweise in der EP-A 83 901 065, DE-A-39 35 098 und US-A-5,057,426 offenbart. In der EP-A 83 901 065 wird zum Beispiel Gamma-Glycidyloxypropyltrimethoxysilan und N, N-Dimethyl aminoethanol zur Modifizierung des Trägermaterials verwendet.

Genügt die nach dem Goldstandard umfassend die Standard-PCR erhaltene PCR-Sensitivität nicht, so wird die deutlich sensitivere nested-PCR (= geschachtelte PCR) durchgeführt, bei der zwei PCR-Reaktionen nacheinander geschaltet werden. Ein Anteil (= Aliquot) des PCR-Produktes aus der ersten Amplifikation dient als Matrize für die zweite PCR. In dieser wird durch ein zweites Primerpaar, das an Sequenzbereiche innerhalb dieser Matrize bindet (nested primer), ein kürzeres DNA-Fragment amplifiziert. Der Vorteil der nested-PCR gegenüber der Standard-PCR ist eine um etwa 2 - 3 Zehnerpotenzen gesteigerte Sensitivität gepaart mit gesteigerter Spezifität, da für das nested-PCR-Produkt nur das Produkt der ersten Amplifikation als Matrize dienen kann. Auch geringste Spuren von DNA können mit dieser Methode nachgewiesen und diagnostischen Zwecken zugänglich gemacht werden. Bei optimaler Einstellung einer nested PCR genügen je nach Zielsequenz und -objekt bereits 1 bis wenige Matrizen zur Amplifikation. Im Vergleich zur Standard-PCR müssen allerdings nachteilhaft zusätzliche Verfahrensschritte durchgeführt werden. Genügt die PCR-Sensitivität einer Probenvorbereitung nach dem Goldstandard mit der Standard-PCR nicht, so wäre dann ein Verfahren wünschenswert, welches eine verbesserte PCR-Sensitivität ermöglicht, ohne den erforderlichen Aufwand steigern zu müssen.

Zwar gibt es alternative Verfahren, die zu guten PCR-Sensitivitäten führen, ohne dafür einen Aufwand betreiben zu müssen, der dem Goldstandard mit einer nested-PCR entspricht. So offenbart beispielsweise die Druckschrift WO 92/17609 A1 ein Verfahren für die Ermittlung von Zielzellen, bei der die Zellen zunächst intakt mit Hilfe von Antikörpern an magnetischen Körnern - bekannt unter der Bezeichnung "magnetische Beads" - gebunden und so angereichert werden. Derartige Verfahren sind jedoch sehr spezifisch und können daher nur relativ begrenzt eingesetzt werden. Die Druckschrift WO 95/13368 offenbart die direkte Amplifikation von auf magnetischen Partikeln kondensierten Nukleinsäuren. Aufgabe der Erfindung ist die Schaffung eines einfachen Verfahrens für die Vorbereitung einer biologischen Probe mit hoher PCR-Sensitivität.

Die Aufgabe wird durch ein Verfahren mit den Merkmalen des Anspruchs 1 und eine Vorrichtung mit den Merkmalen des Anspruchs 10 gelöst. Vorteilhafte Ausgestaltungen ergeben sich aus den Unteransprüchen. Zur Lösung der Aufgabe umfasst ein Verfahren für die Vorbereitung einer biologischen Probe die Schritte Lyse, Binden der Nukleinsäuren an magnetische Partikel, wobei die Bindung über Anionenaustauscher erfolgt, optional Waschen sowie eine zweistufige Amplifizierung. Während der ersten Amplifizierung werden die gesuchten DNA-Stränge relativ unspezifisch amplifiziert. Die so erhaltenen relativ unspezifisch amplifizierten DNA-Stränge werden anschließend spezifisch amplifiziert. Die zweistufige Amplifizierung ist insbesondere eine nested-PCR. Im Unterschied zum Goldstandard entfällt die Elution sowie die sich daran in vielen Fällen anschließende Entsalzung der aus der Elution resultierenden Lösung. Zwar erfordert eine zweistufige Amplifizierung wie die nested-PCR im Vergleich zur Standard-PCR einen zusätzlichen technischen und zeitlichen Aufwand. Dies wird aber beim erfindungsgemäßen Verfahren kompensiert, indem die Elution nebst Entsalzung entfällt. Insgesamt entspricht der erfindungsgemäß erforderliche Aufwand dem Aufwand, der bei der Einhaltung des Goldstandards mit Standard-PCR erforderlich ist. Allerdings kann im Vergleich zum Goldstandard umfassend eine Standard-PCR die PCR-Sensitivität deutlich gesteigert werden. Insgesamt kann daher mit dem erfindungsgemäßen Verfahren das Verhältnis von Aufwand zu Nutzen gesteigert werden. Das erfindungsgemäße Verfahren kann die aus dem Stand der Technik bekannten Schritte und Vorrichtungen umfassen, soweit diese der Durchführung des erfindungsgemäßen Verfahrens nicht entgegenstehen. So können beispielsweise die eingangs genannten Lysepuffer verwendet werden. So kann die Lyse nebst Bindung in bekannter Weise über chaotrope Salze sowie mittels Ethanol an vorzugsweise magnetische Silicapartikel erfolgen. Die Lyse kann mechanisch unterstützt werden, und zwar vorzugsweise durch Ultraschall, oder durch Rühren mit Hilfe eines magnetisch angetriebenen Rührstabs, um schnell und einfach einen Aufschluss mechanisch zu unterstützen.

Anstelle einer PCR können auch andere Amplifikationsverfahren durchgeführt werden, so zum Beispiel WGA (Whole Genome Amplification) oder Reverse Transkription. Bevorzugt wird allerdings eine nested- PCR durchgeführt oder eine nested PCR, bei der die erste PCR eine one-step RT PCR beinhaltet.

Die PCR stellt jedoch für das zweistufige Amplifikationsverfahren das robusteste Verfahren dar. Für den Fachmann ist es prinzipiell möglich, die erste und zweite Nukleinsäureamplifikation auch mit Verfahren durch zuführen, die nicht auf der Verwendung einer hitzestabilen-Polymerase und mehrstufigen Temperaturschritten (also auf PCR) basieren. Derartige Verfahren sind für die Amplifikation von DNA: "Helixdependend Amplification" (HDA); "Recombinase Polymerase Amplfication" (RPA); "Sequence-Specific Rolling Circle Amplification" (RCA); "Loop-mediated Isothermal Amplification" (LAMP). Für die Amplifikation von RNA kann auch alternativ zur einfachen reversen Transkription, Methoden wie "Signal Mediated Amplification of RNA Technology" (SMART) oder "Nucleic acid sequence-based amplification" (NASBA) verwendet werden. Natürlich ist es dem Fachmann auch möglich, diese und andere bekannte Verfahren zur Amplifikation nach Bedarf zu kombinieren. Die erste und zweite Amplifikation können auch als Multiplex-Amplifikationen ausgestaltet sein.

Spezifisches Amplifizieren bedeutet, dass im Idealfall nur ein Amplicon vervielfältigt wird. "Unspezifisches Amplifizieren" bedeutet eine gleichzeitige und ungezielte Amplifikation von mehreren Gensequenzen durch ein unvollständiges Hybridisieren der Primer, d. h. die Primer binden in den ersten Zyklen der PCR auch unvollständig mit "Missmatch" an die Ausgangsnukleinsäure und werden durch die Polymerase verlängert. Diese "falschen" Amplifikate können im Verlauf der PCR wieder als Template fungieren (die Primer binden dann perfekt) und werden dann amplifiziert - dies äußert sich dann in Form von Nebenprodukten in der PCR.

Das Binden erfolgt mittels Anionenaustausch, da bereits während der Lyse gebunden werden kann. Dies trägt dazu bei, dass das Verfahren leicht in einem geschlossen, sehr kleinen mikrofluidischem System durchgeführt werden kann. Nach dem Stand der Technik ist zwar das Binden mittels Anionenaustausch mit dem Nachteil verknüpft, dass die Elution den Einsatz von Salzen mit hoher Konzentration erfordert, was im Anschluss an die Elution eine Entsalzung erforderlich macht. Da erfindungsgemäß die Elution entfällt, entfällt auch eine nachfolgende Entsalzung.

Um die gebundene Nukleinsäure geeignet einer nested-PCR zuführen zu können, wird die gebundene Nukleinsäure in einer Ausführungsform der Erfindung nach dem Binden separiert, indem beispielsweise Partikel, an die die Nukleinsäure gebunden ist, zentrifugiert werden. Der Überstand, also der zuletzt verwendete Puffer bzw. die zuletzt verwendete Lösung, wird verworfen. Wurde beispielsweise zuvor gewaschen, so werden so die Waschpuffer durch abschließendes Verwerfen des Überstandes entfernt. Durch das Separieren wird die PCR-Sensitivität gesteigert. Beispielsweise weise eine Probe 10³ Bakterien oder Viren auf. Nach dem Stand der Technik wird hieraus ein Lysat von typischerweise 1,5 ml Gesamtmenge gewonnen. Hiervon können allerdings nur mit 1 bis 3 Mikroliter eine PCR durchgeführt werden. Dies hat zur Konsequenz, dass nur 0 bis 1 Kopie(n) in eine PCR 1 gelangen. Diese geringe Menge kann zu Sensitivitätsproblemen führen. Durch die erfindungsgemäße Aufkonzentrierung wird erreicht, dass annähernd quantitativ 10³ Kopien der PCR 1 zugeführt werden und so dazu beigetragen wird, die überraschend hohe Sensitivität zu erzielen.

In der Erfindung handelt es sich bei den vorgenannten Körpern um magnetische Partikel (sogenannte "magnetische beads"). Hierdurch lässt sich besonders schnell die gewünschte Separation bzw. Aufkonzentrierung durchführen und zwar beispielsweise mit dafür vorgesehenen aus dem Stand der Technik bekannten Vorrichtungen. Dazu wird beispielsweise ein für die Durchführung der vorhergehenden Schritte verwendetes Röhrchen in eine kommerziell erhältliche Vorrichtung gesteckt. Die daran positionierten Permanentmagneten ziehen die Magnetpartikel an die Wand des Röhrchens. Somit lässt sich sicher der Überstand nach einer solchen Magnetseparation entnehmen. Der Überstand wird wiederum verworfen. Mit den separierten Bestandteilen, die die magnetischen Partikel nebst den daran gebundenen Nukleinsäuren umfassen, wird beispielsweise eine PCR 1, also die erste Stufe einer nested-PCR durchgeführt. Dies trägt dazu bei, dass das Verfahren auf einfache Weise in einem geschlossenen, mikrofluidischem System durchgeführt werden kann. Es befindet sich dann in einer ersten PCR-Kammer ein Magnet, nachfolgend auch Zentralmagnet genannt. Dieser Zentralmagnet ist größer als der Durchmesser von Abflusskanälen oder aber die Abflusskanäle sind durch ein Sieb oder eine Fritte von der ersten PCR-Kammer getrennt. Werden nun in die erste PCR-Kammer die magnetischen Partikel mit den daran gebundenen Nukleinsäuren mit Hilfe einer Flüssigkeit transportiert, so bleiben die magnetischen Partikel, also die magnetischen Beads am Magneten hängen. Wird die Flüssigkeit aus der ersten PCR-Kammer abgesaugt, so gelingt so die gewünschte Separierung. Die Kanaldurchmesser der abführenden Kanäle bzw. die Porenweite der Fritte bzw. des Siebes sind so gewählt, dass der in der Kammer befindliche Zentralmagnet, an den keine Nukleinsäuren gebunden sind, zurückgehalten wird. Da nur ein relativ großer Magnet mit daran haftenden magnetischen Körnern bzw. Beads zurückgehalten werden muss, besteht auch nicht die Gefahr, dass Abflusskanäle oder Siebe nachteilhaft zugesetzt werden können. Grundsätzlich kann das Verfahren aber auch mit nicht magnetischen Körnern bzw. Beads durchgeführt werden, die dann durch ein Sieb in der PCR-Kammer von der Lösung oder Flüssigkeit getrennt werden, mit der die Beads bzw. Körner zuvor in die erste PCR-Kammer hinein transportiert wurden.

Im Anschluss an eine durchgeführte PCR 1 einer nested-PCR wird wieder separiert, was im Fall von eingesetzten magnetischen Partikeln besonders einfach mit Hilfe eines Magneten gelingt. Der Überstand enthält Amplicons, also DNA - Stränge, die durch die PCR 1 vervielfältigt wurden. Ein Aliquot des Überstandes, also ein Teil des Überstandes wird der PCR 2 zugeführt. Hier wird in einer aus dem Stand der Technik bekannten Weise die nested-PCR fortgesetzt.

In einer Ausführungsform der Erfindung wird das Verfahren ganz oder zumindest überwiegend in einem geschlossenen System durchgeführt, welches bereits die benötigten PCR-Reagenzien umfasst und zwar insbesondere in getrockneter Form. Wesentlich ist dann, dass vor allem die nested-PCR bzw. ein äquivalentes zweistufiges Amplifikationsverfahren in einem geschlossenen System durchgeführt wird, was insbesondere dann technisch ohne weiteres störungsfrei realisiert werden kann, wenn die Nukleinsäuren an magnetische Partikel gebunden sind. Zwar ist eine nested-PCR sehr sensitiv, aber die nested-PCR hat jedoch gerade wegen ihrer hohen Sensitivität einen Nachteil - sie ist sehr kontaminationsanfällig. Durch ungewollte Verschleppung von Amplicons und deren erneute Amplifikation kann es leicht zu falsch-positiven Ergebnissen kommen. Um diesen Schwierigkeiten vorzubauen, ist es vorteilhaft, die PCR Reaktion in einem geschlossenen System mit darin enthaltenen PCR-Reagenzien durchzuführen. Es können so falsch-positive Ergebnisse minimiert werden.

Das Verfahren lässt sich sogar in einem mikrofluidischen System durchführen, was dann sogar den Platzbedarf minimiert.

In einer Ausführungsform der Erfindung weist der eingesetzte Lysepuffer einen Salzgehalt von insgesamt weniger als 50 Millimolar, also 50 Millimol/Liter auf. Es hat sich herausgestellt, dass durch Beachtung dieser Grenze die PCR-Sensitivität deutlich gesteigert werden kann.

Der pH-Wert des verwendeten Lysepuffers ist vorzugsweise auf Werte zwischen pH 7,5 bis 9,5 eingestellt. Auch dies steigert die PCR-Sensitivität. Um den pH-Wert in genannter Weise zu stabilisieren, werden vorzugsweise kommerziell erhältliche Puffer Tris, Hepes und/ oder Mops insbesondere mit einer Konzentration von 10 bis 20 Millimolar eingesetzt, um zu guten PCR-Sensitivitäten zu gelangen. Die vorgenannten Bezeichnungen stellen Abkürzungen dar, die dem Fachmann geläufig sind.

Als Detergenzien werden für die Durchführung der Lyse in einer Ausführungsform der Erfindung nichtionische Detergenzien eingesetzt. Geeignete nichtionische Detergenzien werden unter den Markenbezeichnungen Tween®, Nonidet P-40 oder Brij® kommerziell vertrieben. Auch diese Auswahl ermöglicht es, zu guten PCR-Sensitivitäten zu gelangen.

Die Konzentration von nichtionischen Detergenzien beträgt vorzugsweise 0,1 bis 0,4 Vol.-%.

Optional wird zum Lysepuffer Polyvinylpyrrolidon (abgekürzt als: PVP) und zwar insbesondere von 0,05 bis zu 0,1 Vol.-% hinzugefügt. Durch PVP werden Inhibitoren gebunden und so weiter verbessert eine gute PCR-Sensitivität erhalten.

Ein einfaches Verfahren für die Vorbereitung einer biologischen Probe mit einer immer noch relativ hohen PCR-Sensitivität kann auch erreicht werden, indem die durch Aufschluss erhaltene Nukleinsäure an Partikel gebunden wird, die Partikel mit den daran gebundenen Nukleinsäuren separiert werden und die gebundenen Nukleinsäuren amplifiziert werden. Die Amplifikation kann dann auch durch ein klassisches Verfahren wie zum Beispiel einer qPCR erfolgen, also ein Verfahren, bei dem erst nach Ablauf der PCR eine quantitative Auswertung erfolgt. Eine relativ hohe Sensitivität gelingt vor allem dann, wenn der Lysepuffer geeignet gewählt wird. Ein geeignet gewählter Lysepuffer umfasst insbesondere die in Unteransprüchen angegebenen Merkmale eines Lysepuffers. Es können aber auch andere isothermale Amplifikationen durchgeführt werden.

Nachfolgend wird die Erfindung anhand von Beispielen näher erläutert.

Anionenaustauscherpartikel (nämlich RSE Anionenaustausch Beads bzw. Körner) für das Binden von Nukleinsäure wurden wie folgt hergestellt, wobei die Herstellungsgrundlage für diese Partikel in der Patentanmeldung: WO 2007/065933 A1 beschrieben ist:

Als Vorbereitung löst man je 302 ml SPAN 85 in 13 I Norpar 12 und 1,9 I zähflüssiges Paraffin. Anschließend gibt man 112,5 ml EGDMA und 261 ml GMA, die man zuvor von Inhibitoren befreit hat, in eine 2000 ml Nalgene-Flasche oder ein 2000 ml Becherglas, setzt 370 ml Ethylenglykol, 11,25 g AIBN und 279 g Bayoxide E 8706 zu und homogenisiert 2 Minuten bei höchster Stufe am IKA Ultra Turrax. In vier Portionen wird je ein Viertel der Norpar-Lösung in ein 5000 ml Kunststoffgefäß gegeben und das Gefäß an einen Homogenisator angeschlossen. Man setzt bei niedrigster Stufe je ein Viertel einer Eisenoxidsuspension zu und homogenisiert 60 Sekunden lang unter voller Leistung. Dann überführt man jeweils die Emulsion in einen 20 I Reaktor mit Rückflusskühler, KPG Rührer und Gasdurchleitungsrohr bei 250 rpm und steigert die Drehzahl des Rührers auf 300 rpm. Man setzt dann anschließend langsam die Eisenoxidemulsion in organischem Lösungsmittel zu, entgast 5 Minuten durch Stickstoffdurchleiten, beschleunigt auf 350 rpm, und rührt dann über Nacht bei 350 rpm. Man beginnt schon während des Gasdurchleitens mit dem Erhitzen, hält dann eine Stunde auf 70 °C, dann über Nacht auf 80 °C.

Am nächsten Morgen filtriert man die organischen Lösungsmittelmengen über eine Filternutsche mit Polyethylen-Fritte ab und bewahrt die Ölreste auf. Der magnetische Rückstand wird dann dreimal mit Aceton gewaschen, dabei wartet man nach Aceton-Zugabe noch ca. 10 Minuten ab, bevor man absaugt. Dann nimmt man den Rückstand in VE Wasser auf, wäscht dreimal mit Wasser, erneut einmal mit Aceton und zweimal mit abs. Ethanol und trennt dabei die Lösungsmittel mit magnetischer Separation ab, dabei rührt man auf und lässt das Lösungsmittel 5 Minuten einwirken. Beim ersten Wasserwaschschritt lässt man auf die Gefäße für 10 Minuten in einem Ultraschallbad Ultraschall einwirken und setzt dann die Separation fort. Nach dem letzten Wasserschritt sowie den letzten drei Aceton- und Ethanolschritten trennt man die Flüssigkeit gründlich ab, bzw. man lässt die Partikel in der letzten Ethanollösung stehen. Um die Ausbeute zu bestimmen, wird von der letzten Suspension eine Portion abgenommen und zur Bestimmung des Feststoffgehalts getrocknet. Das fertige Produkt weist eine durchschnittliche Teilchengrößenverteilung von 10 µm auf.

Zunächst wird eine 2,5 g enthaltende Polymersuspension in Ethanol in eine Glasfilternutsche, Porosität 4 gegeben und viermal mit wasserfreiem Diglyme bzw. Toluol gewaschen. Man nimmt den Rückstand in 50 ml einer knapp 10%igen Lösung von Bis-Tris bzw. Diisopropylamino-ethylamin in wasserfreiem Diglyme bzw. Toluol auf und überführt in einen 100 ml Dreihalskolben. Dann hängt man an den Kolben einen Rückflusskühler, entgast kurz zweimal und lässt dann bei 100 rpm und 120°C über Nacht reagieren (16h). Am nächsten Morgen wäscht man die Polymere viermal mit voll entsalztem Wasser, dreimal mit Ethanol und bewahrt die Polymere dann unter Ethanol auf.

Eine weitere Sorte an funktionalisierten Anionenaustauscher-Magnetpartikel (AX1) wird wie folgt synthetisiert.

Man setzt eine Suspension von MagAttract "G" ein (aus BioSprint DNA Blood Kit 96, QIAGEN, Artikel # 940057) und wäscht viermal mit Millipore-Wasser. Die Überstände werden dabei mittels magnetischer Separation abgetrennt und verworfen. Anschließend werden 2,5 g der getrockneten magnetischen Partikel in 25 ml trockenem γ-Glycidoxy-propyl-triethoxysilan (Aldrich, Artikel-# 440167)resuspendiert. Dann entgast man kurz am Rotationsverdampfer und belüftet mit Stickstoff. Anschließend erhitzt man das Bad auf 140 °C und lässt die Probe acht Stunden am Rotationsverdampfer reagieren. Das Produkt wird dann magnetisch separiert und die Überstände viermal mit trockenem Dioxan gewaschen. Dann wird das magnetische Kieselgel in 25 ml trockenem Dimethylaminoethanol suspendiert und mit 250 µl Bortrifluorid-etherat versetzt. Anschließend wird das Produktgemisch am Rotationsverdampfer 24 Stunden unter Rückfluss erhitzt und nach dem Abkühlen magnetisch separiert, wobei man die Überstände verwirft. Dann wäscht man mehrfach mit Dioxan, Methanol und Diethylether und trocknet bei 50 °C im Vakuumtrockenschrank.

Um die Sensitivität des erfindungsgemäßen Verfahrens zu ermitteln, wird bei den nachfolgenden Beispielen als PCR 2 eine Real-Time-PCR durchgeführt, mit der die gewonnene DNA quantifiziert wird.

Die Real-Time- PCR ist eine Vervielfältigungsmethode für Nukleinsäuren, die auf dem Prinzip der herkömmlichen Polymerase-Kettenreaktion (PCR) beruht, und zusätzlich die Quantifizierung der gewonnenen DNA ermöglicht. Die Quantifizierung wird mit Hilfe von Fluoreszenz-Messungen durchgeführt, die während eines PCR-Zyklus erfasst werden. Die Fluoreszenz nimmt proportional mit der Menge der PCR-Produkte zu. Am Ende eines Laufs (der aus mehreren Zyklen besteht) wird anhand von erhaltenen Fluoreszenzsignalen die Quantifizierung in der exponentiellen Phase der PCR vorgenommen. Nur in der exponentiellen Phase der PCR (die wenige Zyklen in einem Lauf dauert) ist die korrekte Quantifizierung möglich, da während dieser Phase die optimalen Reaktionsbedingungen herrschen. Diese Methode unterscheidet sich somit von anderen quantitativen PCR-Methoden (qPCR), die erst nach Ablauf der PCR eine quantitative Auswertung (z.B. kompetitive PCR), meist unter Einbeziehung einer gelelektrophoretischen Auftrennung der PCR-Fragmente, vornehmen.

In der ersten Phase der Amplifikation einer PCR ist die Templatemenge begrenzt und die Wahrscheinlichkeit, dass sich Template, Primer und Polymerase treffen, suboptimal, während in der dritten Phase der Amplifikation die Menge der Produkte (DNA, Pyrophosphat, Monophosphatnucleotide) derart ansteigt, dass es zur Hemmung durch diese kommt, häufiger Produktfragmente miteinander hybridisieren, die Substrate langsam verbraucht werden und letztlich die Polymerasen und Nucleotide durch die Hitze langsam zerstört werden. Ein exponentieller und daher quantifizierbarer Anstieg findet sich nur in der Phase dazwischen. Exponentiell bleibt eine PCR bei 12 bis 400 Ausgangskopien für ca. 30 Zyklen, bei 200 bis 3200 für 25 Zyklen und bei anfänglich 3200 bis 51200 für höchstens 20 Zyklen. Um immer am Anfang der exponentiellen Phase messen zu können, wird häufig der CT-Wert (Threshold Cycle = "Schwellenwert-Zyklus") bzw. der Cp-Wert (Crossing Point) verwendet, der den Zyklus beschreibt, an dem die Fluoreszenz erstmalig signifikant über die Hintergrund-Fluoreszenz ansteigt.

Im Rahmen eines ersten Ausführungsbeispiels wurde das Bakterium *Escherichia coli* in Blut verdünnt und untersucht. Versuche wurden mit den genannten unterschiedlichen Anionenaustausch-Beads durchgeführt, um zu untersuchen, ob die Wahl der Anionenaustausch-Beads mit unterschiedlicher Oberflächenmodifikation das Ergebnis beeinflusst.

Folgende Materialien wurden eingesetzt:
AX1 Anionenaustausch Beads
RSE Anionenaustausch Beads
Blut aus Blutbeutel
Lyse Buffer GE (01% PVP MW 10.000; 0,45% Tween-20; 0,45% NP-40; 10 mM Tris/Cl pH 7,5; 1 mM EDTA)
*Escherichia coli* O157:H7 (ATTC 700728; Apathogener Stamm) Verdünnungen: 10⁸/ml; 10⁷/ml, 10⁶/ml; 10⁵/ml
PCR-Primer
   EOF: ATGCTACCCCTGAAAAACTC
   EOR: CGCTTGAACTGATTTCCTC
   EFwd: CGATGATGCTACCCCTGAAAAACT
   ERev: TATTGTCGCTTGAACTGATTTCCTC
   EPro: "6-Fam"-CGTTGTTAAGTCAATGGAAAACCTG- BHQ1

Der Versuch wurde nach dem folgenden Protokoll durchgeführt:

In verschiedene 1,5 ml Microtubes, also in kleine, röhrenförmige Gefäße werden jeweils 100 µl Blut und 10 µl einer verdünnten E.coli Übernachtkultur gefüllt. Die Verdünnungen waren 10⁸/ml; 10⁷/ml; 10⁶/ml; 10⁵/ml. In die Microtubes wurden 1 ml Buffer GE und 20 µl Proteinase K und 10 µl Lysozym (10 mg/ml) eingefüllt.

In die Microtubes wurden 20 µl Anionenaustausch-Magnetpartikel (50 mg/ml) hinzugefügt und zwar je nach Ansatz entweder AX1 oder RSE Magnetpartikel. Es wurde anschließend eine Inkubation durchgeführt, indem die Microtubes für 30 min auf einem Eppendorf Thermomixer bei 56°C und 1400 Umdrehungen pro Minute behandelt wurden. Danach wurde die Inkubation fortgesetzt und zwar für 10 min auf dem Eppendorf Thermomixer bei 80°C und 1400 Umdrehungen pro Minute. Durch diese Behandlung wird die Probe aufgeschlossen und gebunden.

Es werden dann die Röhrchen in eine Magnetseparationsvorrichtung gesteckt. Für 20 sec lässt man die Magnetpartikel sich an der Wand des Röhrchens durch die Einwirkung des Magnetfeldes der Permanentmagnete der Vorrichtung abscheiden. Nun wird der Überstand abpipettiert und verworfen. Anschließend werden die Magnetpartikel mit Buffer GE zweimal gewaschen. Hierzu werden je 800 µl Buffer GE in das Röhrchen pipettiert und das Magnetpartikelsediment durch kurzes vortexen resuspendiert. Als nächstes wird eine Magnetseparation durchgeführt und der Überstand erneut verworfen.

Nun wird das Magnetpartikelsediment durch kurzes Vortexen in 100µl BiDest., also zweifach destilliertes Wasser resuspendiert und in ein PCR-Röhrchen überführt. Der Überstand wird sorgfältig und quantitativ entfernt. Die magnetischen Beads werden mit 50 µl PCR Lösung resuspendiert und die erste PCR wird gestartet und wie folgt durchgeführt:

Es werden 25 µl Hot Star Tag Master Mix (QIAGEN), 1 µl EOF (100 µM), 1 µl EOR (100 µM) sowie 23 µl Wasser zugegeben. Nach erstmaliger Aktivierung der Taq Polymerase durch 15 minütige Inkubation bei 95°C werden insgesamt 20 Temperatur-Zyklen durchgeführt. Ein Zyklus umfasst die nachfolgenden Schritte:
Denaturierung 15 s; 95°C
Annealing 30 s; 52°C
Extension 15 s; 72°C.

Nach der PCR1 wird das PCR- Röhrchen auf ein Magnetseparations-Gestell gesetzt und 5 µl Überstand in ein frisches PCR Tube transferiert.

Die zweite PCR, nämlich eine quantitative Real Time PCR, wird mit einer Zugabe von 20 µl PCR 2 - Reagenzien begonnen. Folgende Reagenzien werden eingesetzt:

12,5 µl QIAGEN QuantiTect Probe PCR Mastermix, 0,1 µl EFwd; 0,10 µl ERev; 0,05 µl EPro sowie 7,25 µl bidest. Wasser. Nach der Taq Polymerase Aktivierung für 5 min bei 95°C, werden insgesamt 40 Zyklen durchgeführt. Ein Zyklus umfasst die folgenden Schritte: [Denaturierung 15 s; 95°C; Extension 60 s; 60°C).

Als Vergleichsversuch wurde eine Vorbereitung nach dem Goldstandard mit einer Standard-PCR mit gemäß dem kommerziell erhältlichen "QIAamp DNA Blood Mini Kit" durchgeführt. Es wurden 10 µl E. coli Verdünnung zusammen mit 100 µl Blut nach dem Standard Protokoll präpariert und mit 100 µl Elutionspuffer eluiert.

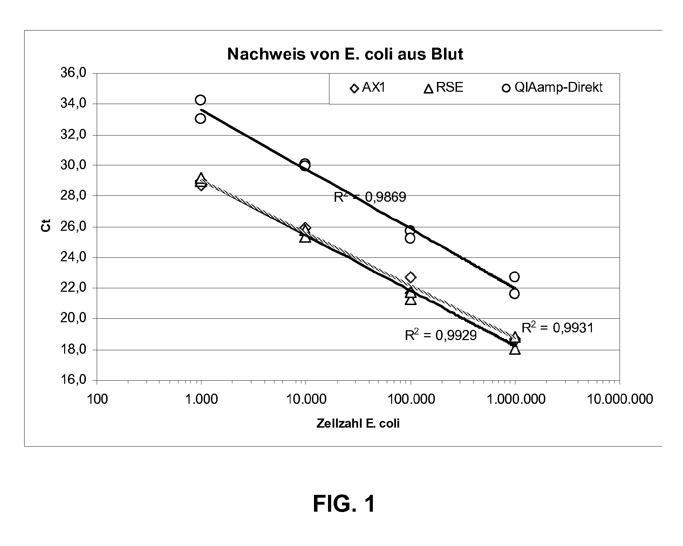

Das Ergebnis der quantitativen PCR zum Nachweis von *E*. *coli*-DNA gemäß Beispiel 1 mit unterschiedlichen Anzahlen von Bakterien in der Präparation wird in Figur 1 gezeigt und mit dem Ergebnis verglichen, das durch die Standard Präparation mit Elution und Standard-PPCR erhalten wurde. Die erfindungsgemäß erhaltenen ct-Werte lagen um ca. zwei Einheiten unterhalb der ct-Werte, die aufgrund des Standard-Verfahrens erhalten wurden. Da die Sensitivität umso besser ist, je niedriger der ct-Wert ist, wurde also trotz vergleichbaren Aufwandes die Sensitivität durch das erfindungsgemäße Verfahren verbessert. Die Versuchsreihen verdeutlichen ferner, dass sich mit dem erfindungsgemäßen Verfahren eine gute Linearität erzielen lässt, die den dynamischen Bereich des PCR Nachweissystems wiederspiegelt. Auch in dieser Hinsicht wurde also ein sehr gutes Ergebnis erzielt. Schließlich verdeutlicht das Ergebnis, dass das Ergebnis nicht maßgeblich von der Wahl der Anionenaustausch-Beads abhängt.

Im Rahmen eines zweiten Ausführungsbeispiels wurde das Bakterium *Eschrichia coli* in SurePath Medium verdünnt und wie folgt erfindungsgemäß sowie - zwecks Vergleich - gemäß dem Stand der Technik vorbereitet und untersucht.

Verwendetes Material:
AX1 Anionenaustausch Beads
RSE Anionenaustausch Beads
Blut aus Blutbeutel
Lyse Buffer GE (01% PVP MW 10.000; 0,45% Tween-20; 0,45% NP-40;
10 mM Tris/Cl pH 7,5; 1 mM EDTA)
Escherichia coli O157:H7 (ATTC 700728; Apathogener Stamm) Verdünnungen: 10⁸/ml; 10⁷/ml; 10⁶/ml; 10⁵/ml
PCR-Primer
   EOF: ATGCTACCCCTGAAAAACTC
   EOR: CGCTTGAACTGATTTCCTC
   EFwd: CGATGATGCTACCCCTGAAAAACT
   ERev: TATTGTCGCTTGAACTGATTTCCTC
   EPro: "6-Fam"-CGTTGTTAAGTCAATGGAAAACCTG- BHQ1

Gemäß dem folgenden Protokoll wurde vorbereitet:
- In ein 1,5 ml Microtube werden 100 µl Blut und 10 µl E.coli Übernachtkultur in verschieden Verdünnungen gegeben
- Zugabe 1 ml Buffer GE und 20 µl Proteinase K und 10 µl Lysozym (10 mg/ml)
- Zugabe 20 µl MagBeads
- Inkubation: 30 min auf Eppendorf Thermomixer bei 56°C und 1400 rpm
- Inkubation: 10 min auf Eppendorf Thermomixer bei 80°C und 1400 rpm
- Magnetseparation und Verwerfen des Überstandes
- Mag Beads 2X mit Buffer GE waschen (je 800 µl; kurz vortexen; Magnetseparation und Verwerfen des Überstandes
- MagBead Sediment in 100µl BiDest Wasser resuspendieren und in ein PCR Tube überführen
- Überstand sorgfältig und quantitativ entfernen
- Beads mit 50 µl PCR Lösung resuspendieren und PCR1 starten
   **Nested PCR1**
   25 µl Hot Star Tag Master Mix (QIAGEN)
   1 µl EOF (100 µM)
   1 µl EOR (100 µM)
   23 µl Wasser
   Taq Aktivierung 15 min; 95°C
   Zyklus:
   Denaturierung 15 s; 95°C
   Annealing 30 s; 52°C
   Extension 15 s; 72°C
   Zyklenzahl 20
- Nach der PCR: PCR Tube (also ein PCR-Röhrchen) auf Magnetseparations-Gestell stellen und 5 µl in ein frisches PCR Tube transferieren
- Durchführung PCR2 (quantitative Real Time PCR) durch Zugabe von 20 µl PCR 2 Reagenzien:
   12,5 µl QIAGEN QuantiTect Probe PCR Mastermix, 0,1 µl EFwd; 0,10 µl ERev; 0,05 µl EPro, 7,25 µl bidest. Wasser; Cycling: Taq Aktivierung 5 min; 95°C; 40 Zyklen [Denaturierung 15 s; 95°C; Extension 60 s; 60°C]
   Kontrollversuch nach dem Stand der Technik: QIAamp [10 µl E. coli Verdünnung in 100 µl Blut wurden nach Standard Protokoll präpariert und mit 100 µl eluiert]. "QIAamp Nested:" 5 µl des Eluats wurden in die PCR1 eingesetzt und davon wieder 5µl in die PCR2; "QIAamp Direkt 5 µl des Eluats wurden in die PCR2 eingesetzt.

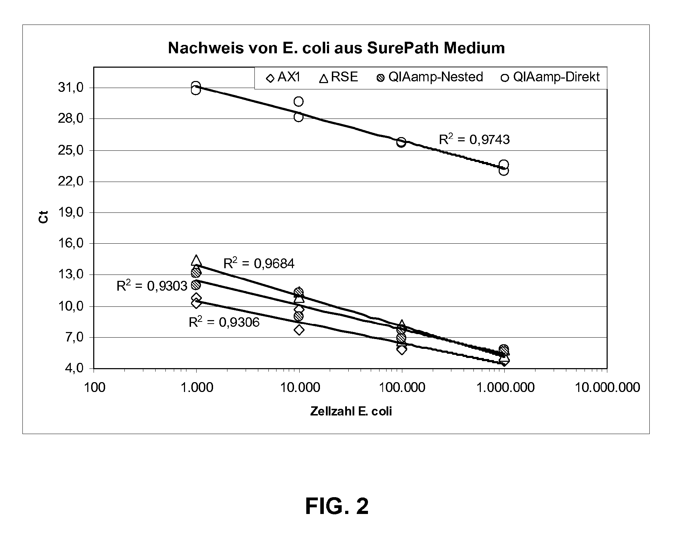

Figur 2 zeigt das Ergebnis der quantitativen PCR zum Nachweis von E. coli-DNA aus Beispiel 2 mit unterschiedlichen Anzahlen von Bakterien in der Präparation. An dem so erzielten Ergebnis überrascht, dass sogar die Sensitivität erreicht wurde, die durch eine Probenvorbereitung nach dem Goldstandard mit einer nested-PCR (QIAamp nested) erzielt wurde. Auch wurde wiederum eine große Linearität erhalten. Bestätigt wurde ferner das Ergebnis aus Beispiel 1, dass die Sensitivität gegenüber dem Standard-Verfahren mit der Standard-PCR (QIAamp direkt) deutlich gesteigert werden konnte, ohne dafür den Aufwand vergrößern zu müssen.

Im Rahmen eines dritten Ausführungsbeispiels wurden SIHA-Zellen in SurePath Medium vorbereitet und untersucht.

Als Materialien wurden eingesetzt:
• Kultivierte SIHA Zellen (humane Zervixkarzinomzelllinie; 2 - 3 Kopien HPV16/Zelle; ATCC: HTB 35 ).
Die Zellen wurden mittels Standard-Zellkulturprotokollen in Zellkulturflaschen kultiviert. Nach dem Waschen mit PSB wurden die Zellen trypsinisiert und die Zellzahl unter dem Mikroskop bestimmt. Jeweils 10⁶ Zellen wurden aliquotiert und durch Zentrifugation sedimentiert. Nach dem Entfernen des Überstandes wurden die Zellen als Sediment eingefroren. Zur Präparation wurden die Zellsedimente durch Zugabe von SurePath Medium resuspendiert. Weitere Verdünnungen wurde jeweils mit SurePath Medium hergestellt.
• AX1 Anionenaustausch Beads
• RSE Anionenaustausch Beads
• Lyse Buffer GE (01% PVP MW 10.000; 0,45% Tween-20; 0,45% NP-40; 10 mM Tris/Cl pH 7,5; 1 mM EDTA)
PCR-Primer

| | |
|---|---|
| 16oFWD | CACCAAAAGAGAACTGCAATG |
| 16iFWD | GGAGCGACCCAGAAAGTTACCAC |
| 16oREV | GGATTCCCATCTCTATATACTA |
| 16iREV | GCATAAATCCCGAAAAGCAAAGTCA |
| 16PRO | AGAATGTGTGTACTGCAAGCAACAG[BHQ-FAM] |

Gemäß dem folgenden Protokoll wurde vorgegangen:
- In ein 2,2 ml Microtube werden 500 µl SurePath Medium mit SIHAs Zellen in verschieden Verdünnungen gegeben
- Zugabe 1 ml Buffer GE und 20 µl Proteinase K
- Zugabe 20 µl MagBeads
- Inkubation: 30 min auf Eppendorf Thermomixer bei 56°C und 1400 rpm
- Inkubation: 10 min auf Eppendorf Thermomixer bei 80°C und 1400 rpm
- Magnetseparation und Verwerfen des Überstandes
- Mag Beads 2X mit Buffer GE waschen (je 800 µl; kurz vortexen; Magnetseparation und Verwerfen des Überstandes
- MagBead Sediment in 100µl BiDest Wasser resuspendieren und in ein PCR Tube überführen
- Überstand sorgfältig und quantitativ Entfernen
- Beads mit 50 µl PCR Lösung resuspendieren und PCR1 starten
   Nested PCR 1 :
   25 µl Hot Star Tag Master Mix (QIAGEN)
   1 µl 16oFWD (100 µM)
   1 µl 16oREV (100 µM)
   23 µl Wasser
   Cycling
   Taq Aktivierung 15 min; 95°C
   Denaturierung 15 s; 95°C
   Annealing 30 s; 52°C
   Extension 15 s; 72°C
   Zyklenzahl 20
- Nach der PCR1: PCR Tube auf Magnetseparations Rack stellen und 5 µl in ein frisches PCR Tube transferieren
- Durchführung PCR2 (quantitative Real Time PCR) durch Zugabe von 20 µl PCR 2 Reagenzien:
   12,5 µl QIAGEN QuantiTect Probe PCR Mastermix, 0,1 µl 16iFWD; 0,10 µl 16iRev; 0,05 µl 16iPro, 7,25 µl bidest. Wasser; Cycling: Taq Aktivierung 5 min; 95°C; 40 Zyklen [Denaturierung 15 s; 95°C; Extension 60 s; 60°C]
   Vergleichsversuche: QIAamp [500µl SurePath Medium mit SIHAs Zellen nach Standard QIAamp Protokoll präpariert und mit 100 µl eluiert]. "QIAamp Nested" 5 µl des Eluats wurden in die PCR1 eingesetzt und davon wieder 5µl in die PCR2;

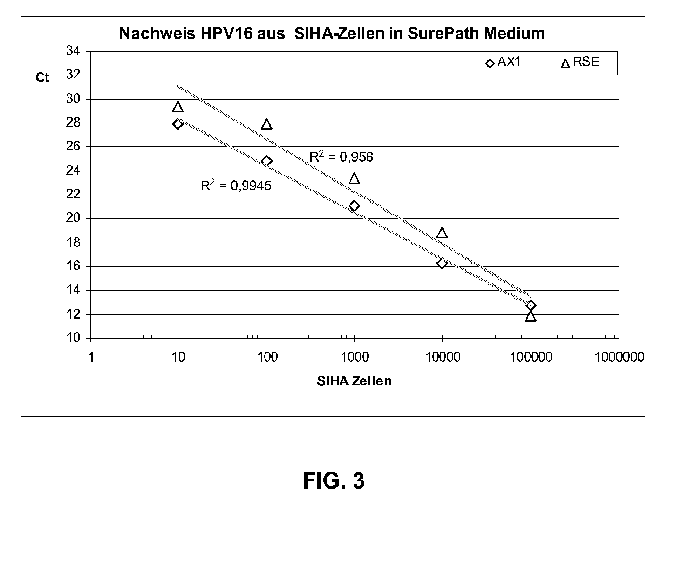

Figur 3 zeigt das Ergebnis der quantitativen PCR zum Nachweis HPV16 aus SIHA Zellen aus Ausführungsbeispiel 3 mit unterschiedlichen SIHA-Zellzahlen in der Präparation.

Fazit: Die Strategie des "Nested PCR Bead Cycling" zeigt für die Präparation von HPV16 aus SurePath Medium eine hohe Linearität des Nachweises von eingesetzten Zellen. Es lassen sich bis zu 100 Zellen sicher nachweisen. Somit eignet sich das erfindungsgemäße Verfahren auch zur HPV Diagnostik.

Im Rahmen eines vierten Ausführungsbeispiels wurde die Bindung und Amplifikation mittels Silika-MagBeads durchgeführt.

Als Materialien wurden eingesetzt:
MasG: MagAttract Suspension G; QIAGEN
MasB: MagAttract Suspension B; QIAGEN
SurePath Medium (BD)
Lyse Buffer G (3 M GITC; 20% NP-40)
Escherichia coli O157:H7 (ATTC 700728; Apathogener Stamm) Verdünnungen: 10⁸/ml; 10⁷/ml; 10⁶/ml; 10⁵/ml
PCR-Primer
   EOF: ATGCTACCCCTGAAAAACTC
   EOR: CGCTTGAACTGATTTCCTC
   EFwd: CGATGATGCTACCCCTGAAAAACT
   ERev: TATTGTCGCTTGAACTGATTTCCTC
   EPro: "6-Fam"-CGTTGTTAAGTCAATGGAAAACCTG- BHQ1

Nach dem folgenden Protokoll wurde vorgegangen:
- In ein 1,5 ml Microtube werden 500 µl SurePath Medium und 10 µl E.coli Übernachtkultur in verschieden Verdünnungen gegeben
- Zugabe 500µl Buffer G und 20µl Proteinase K (QIAGEN)
- Inkubation: 20min 56°C
- Zugabe 500µl Isopropanol und 20µl Silika Magnetpartikel (MasG bzw. MasB)
- 10 min Schütteln auf Eppendorf Thermomixer bei 1400 rpm
- Magnetseparation und Verwerfen des Überstandes
- MagBead Sediment in 100µl Buffer AW2 (QIAGEN) resuspendieren und in ein PCR Tube überführen
- Magnetseparation und Verwerfen des Überstandes
- Spülen der Bead unter separierten Zustand mit 3 x 150 µl Bi Dest Wasser
- Überstand sorgfältig und quantitativ Entfernen
- Beads mit 50 µl PCR Lösung resuspendieren und PCR1 starten
   Nested PCR 1 :
   25 µl Hot Star Tag Master Mix (QIAGEN)
   1 µl EOF (100 µM)
   1 µl EOR (100 µM)
   23 µl Wasser
   Taq Aktivierung 15 min; 95°C
   Zyklus:
   Denaturierung 15 s; 95°C
   Annealing 30 s; 52°C
   Extension 15 s; 72°C
   Zyklenzahl 20
- Nach der PCR1: PCR Tube auf Magnetseparations Rack stellen und 5 µl in ein frisches PCR Tube transferieren
- Durchführung PCR2 (quantitative Real Time PCR) durch Zugabe von 20 µl PCR 2 Reagenzien:
   12,5 µl QIAGEN QuantiTect Probe PCR Mastermix, 0,1 µl EFwd; 0,10 µl ERev; 0,05 µl EPro, 7,25 µl bidest. Wasser; Cycling: Taq Aktivierung 5 min; 95°C; 40 Zyklen [Denaturierung 15 s; 95°C;
   Extension 60 s; 60°C]

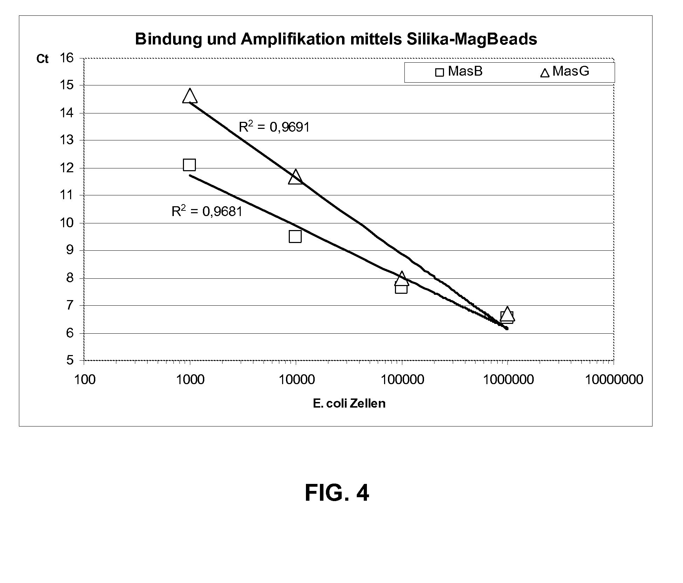

Figur 4 verdeutlicht das Ergebnis der quantitativen PCR zum Nachweis von *E*. *coli*-DNA aus Ausführungsbeispiel 4 mit unterschiedlicher Anzahl von Bakterien in der Präparation. Neben den Einsatz von Anionenaustausch-Magnetpartikeln wurde auch gezeigt, dass das erfinderische Verfahren auch prinzipiell mit Silica-Magnetpartikeln und chaotroper Nukleinsäurebindung durchgeführt werden kann.

Im Rahmen eines fünften Ausführungsbeispiels sollte die RNA Isolierung und deren Nachweis anhand der RNA Bakteriophagen "fr" ermittelt werden.

Hierzu wurde 500 µl Phage fr Suspension [5*10⁶ PFU (plaque forming units)] und 1 ml Lysepuffer GE (01% PVP MW 10.000; 0,45% Tween-20; 0,45% NP-40; 10 mM Tris/Cl pH 7,5; 1 mM EDTA), 20µl Proteinase K und 20µl AX01 Beads in einem 1,5 ml Microtube vermischt.

Zunächst wird das Reagenzgefäß für 15 min auf Eppendorf Thermomixer bei 56°C und 1400 rpm inkubiert. Nach einer Magnetseparation wird der Überstand verworfen und die Magnetpartikel werden zweimal mit Buffer GE gewaschen (je 800 µl; kurz vortexen; Magnetseparation und Verwerfen des Überstandes. Nun wird das Magnetpartikel Sediment in 1x QuantiTect Probe RT-PCR Master Mix (QIAGEN GmbH) resuspendiert und in ein PCR Tube überführt. Nach einer weiteren Magnetseparation wird abermals der Überstand quantitativ entfernt und die Reaktion durch Zugabe und Resuspendieren des Sediments in 50 µl RT-PCR Lösung gestartet.

Der RT-PCR Mix wird wie folgt zusammen gesetzt:
- 25µl 2x QuantiTect Probe RT-PCR Master Mix
- 0.5µl FOF (CTCGAAGTTTACCAATCAAT; 10µM)
- 0.5µl FOR (TATTTATCTGACCACAACGG; 10µM)
- 0.5µl RT Mix
- 23.5µl Wasser

Zur RT-PCR wurden folgende Temperatureinstellungen benutzt
Reverse Transcription 35 min; 50°C
Taq Aktivierung 15 min; 95°C
Zyklus:
Denaturierung 30 s; 95°C
Annealing 60 s; 52°C
Extension 60 s; 72°C
Zyklenzahl 40

Nach der RT PCR wurden 4µl Überstand der 50µl Reaktion auf ein 2%-iges Agarosegel aufgetragen und elektrophoretisch aufgetrennt. Nach einem Anfärben des Gel mit Ethidium-Bromid wurde auf einer Geldokumentationsvorrichtung eine Aufnahme gemacht.

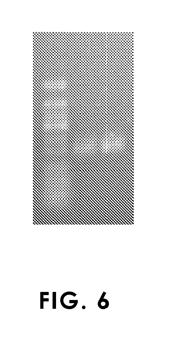

Figur 6 zeigt auf dem Agarosegel (2%-ig), dass beide Proben der fr-Phagen Isolierung eine spezifische Bande (mittlere und rechte Spur) mit der erwarteten Größe (202bp) aufweisen. Somit kann diese Amplifikation basierend auf RNA als Ausgangsmaterials als erfolgreich bewertet werden. Eine einzige RT-PCR kann nach der RNA Präparation und Amplifikation 5*10⁶ pfu Phage fr erfolgreich nachweisen. (Die linke Spur zeigt den DNA Längen Standard)

Diese und das sechste Ausführungsbeispiel zeigen deutlich, dass das erfindungsgemäße Verfahren zur Aufreinigung (Isolierung) und Amplifikation von Nukleinsäuren (wie oben im Detail beschrieben) neben der Isolierung und Amplifikation von DNA ebenso gut zur Isolierung und Amplifikation von RNA eingesetzt werden kann. Wir verweisen auf die obige Offenbarung.

Im Rahmen eines sechsten Ausführungsbeispiels wurden zur Isolierung von RNA HeLa-Zellen eingesetzt.

Als Materialien wurden eingesetzt:
- Kultivierte HeLa Zellen (humane Zervixkarzinomzelllinie). Die Zellen wurden mittels Standard-Zellkulturprotokollen in Zellkulturflaschen kultiviert. Nach dem Waschen mit PSB wurden die Zellen trypsinisiert und die Zellzahl unter dem Mikroskop bestimmt.
- AX1 Anionenaustausch Beads
- Lyse Buffer GE (01% PVP MW 10.000; 0,45% Tween-20; 0,45% NP-40; 10 mM Tris/Cl pH 7,5; 1 mM EDTA)

Gemäß dem folgenden Protokoll wurde vorgegangen:
- In ein 2,2 ml Microtube werden 500 µl 10⁵, bzw. 10³ HeLa Zellen in PBS mit 1 ml Buffer GE, 20 µl Proteinase K und 20 µl AX1 MagBeads vermischt.
- Inkubation: 15 min auf Eppendorf Thermomixer bei 56°C und 1400 rpm
- Magnetseparation und Verwerfen des Überstandes
- Mag Beads 2X mit Buffer GE waschen (je 800 µl; kurz vortexen; Magnetseparation und Verwerfen des Überstandes
- MagBead Sediment in 100µl BiDest Wasser resuspendieren und in ein PCR Tube überführen
- Überstand sorgfältig und quantitativ entfernen
- Beads mit 50 µl RT-qPCR Lösung resuspendieren und PCR in einem Real Time Cycler starten

Zur RT-qPCR wurden folgende Temperatureinstellungen benutzt
- 25µl 2x QuantiTect Probe RT-PCR Master Mix
- 0.5µl Fwd Primer (Humanes Lamin A;
   GGCGGGTGGATGCTGAGAACA; 10µM)
- 0.5µl Rev Primer (Humanes Lamin A;
   TGTCAATCTCCACCAGTCGGG; 10µM)
- 0,25µl Probe (Humanes Lamin A;
   ATCTACAGTGAGGAGCTGCGTGAGA (5'-Fam; 3'-BHQ1;10µM)
- 0.5µl RT Mix
- 23.25µl Wasser

Zur RT-PCR wurden folgende Temperatureinstellungen benutzt
Reverse Transcription 30 min; 50°C
Taq Aktivierung 15 min; 95°C
Zyklus:
Denaturierung 15 s; 94°C
Annealing/ Extension 60 s; 60°C
Zyklenzahl: 40

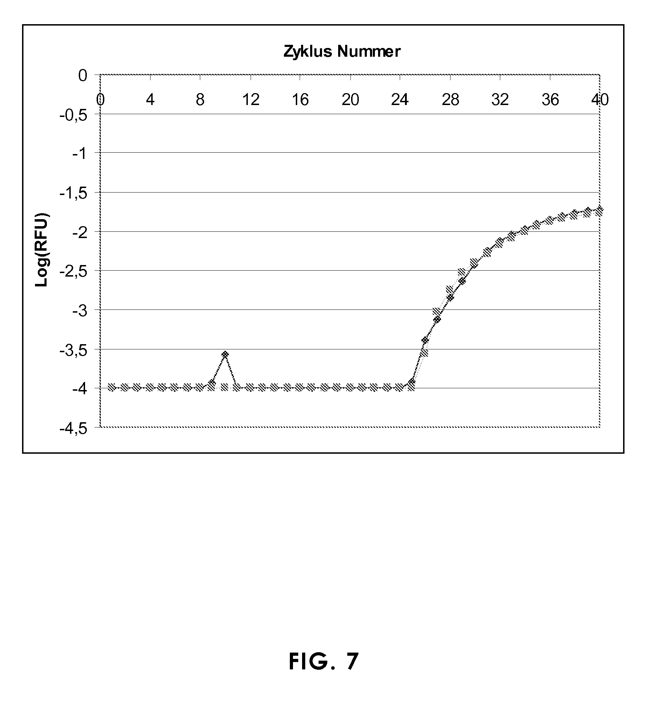

Figur 7 zeigt einen "Amplification Plot" einer Real Time PCR. Die Kurve zeigt den Anstieg der Fluoreszenz zweier RT QPCR Amplifikation von HeLa RNA Präparationen in Anhängigkeit zur Nummer des PCR Zyklus und damit deutliche Amplifikationssignale, die beweisen, dass mit dem beschriebenen Verfahren aus HeLa Zellen RNA isoliert wurde, denn die Primer/Probe Sequenzen des amplifizierten Lamin A Gens sind so gewählt, dass wegen Exon/intron Strukturen des Gens nur ausgehend von einer gespleißten RNA eine Amplifikation starten kann. Das Bespiel zeigt zudem auch noch das unter bestimmte Umständen auch auf eine zweite Amplifikation verzichtet werden kann.

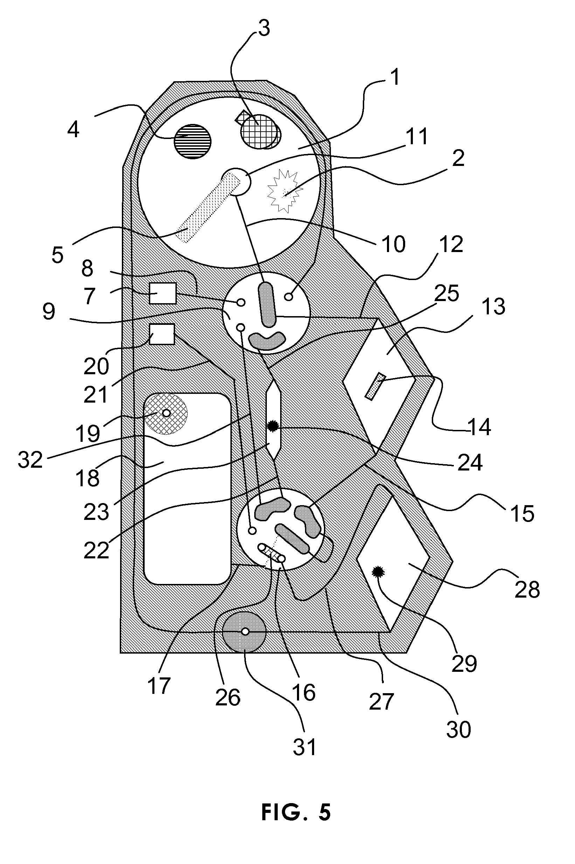

Das Verfahren kann leicht in einem geschlossenen System durchgeführt werden, um so Kontaminationen zu vermeiden. In Figur 5 wird ein Beispiel für ein geschlossenes System zur Durchführung des Verfahrens im Schnitt dargestellt. Die dargestellte Vorrichtung umfasst eine Lysekammer 1, in der sich bereits magnetische Beads (nebst Proteinase K) 2 für das Binden von Nukleinsäure befinden. Die Lysekammer 1 weist eine verschließbare Öffnung 3 auf, über die eine biologische Probe in die Lysekammer 1 gebracht wird. Die Lysekammer 1 ist mit einem Druckausgleichsventil 4 versehen, um den in der Lysekammer 1 herrschenden Druck regulieren zu können, bzw. durch Überdruck dies bei Bedarf zu entleeren. In der Lysekammer befindet sich ein stabförmiger Magnet 5, mit der die Lyse mechanisch unterstützt werden kann. Rotiert der Magnet schnell genug, so lösen sich die magnetischen Beads mit den gebundenen Nukleinsäuren und können weiter geleitet werden. Besonders einfach kann die Lyse aber auch in diesem Fall mit Hilfe von Ultraschall mechanisch unterstützt werden.

In der Lysekammer 1 wird die Probe aufgeschlossen, indem ein Lysepuffer über einen Anschluss 7, einen Kanal 8, eine Ventileinheit 9, einen Kanal 10 von unten über eine Öffnung 11 in die Lysekammer gepumpt wird. Die durch den Lysepuffer enzymatisch und unter Rühren aufgeschlossene Probe bindet an die magnetischen Beads durch Anionenaustausch. Nach dem Binden werden der Lysepuffer zusammen mit den magnetischen Beads und den daran gebundenen Nukleinsäuren über die Öffnung 11 abgesaugt und durch den Kanal 10 zu der Ventileinheit 9 gepumpt und von hier aus über einen Kanal 12 in eine erste PCR-Kammer 13 geleitet. In der ersten PCR-Kammer 13 befindet sich ein kleiner Permanentmagnet 14, an den sich die magnetischen Beads 2 anheften. Der Lysepuffer wird über einen Kanal 15, eine Ventileinheit 16, einen Kanal 17 in eine Abfallkammer 18 weitergeleitet. Die Abfallkammer 18 weist eine nach außen führende Öffnung 19 auf, um so das Entstehen eines Überdrucks in der Abfallkammer 18 zu vermeiden. Die Verbindung 19 kann ein Ventil sein oder in der einfachsten Ausführung eine semipermeable Membran (z.B. GoreTex™).

Über den Anschluss 7 werden nun Waschpuffer durch den Kanal 8, die Ventileinheit 9, den Kanal 12 in die erste PCR-Kammer eingeleitet und über den Kanal 15, die Ventileinheit 16, den Kanal 17 in die Abfallkammer 18 weitergeleitet, um so die magnetischen Beads mit den daran gebundenen Nukleinsäuren zu waschen. Schließlich wird die weitere Zufuhr von Waschpuffern gestoppt und die Waschpuffer aus der ersten PCR-Kammer herausgepumpt. In das System wird nun Wasser über einen Anschluss 20 eingeleitet. Das Wasser gelangt über einen Kanal 21 in die Ventileinheit 16 und wird von dieser über einen Kanal 22 in eine Reagenzienkammer 23 eingeleitet. In der Reagenzienkammer befinden sich getrocknete Reagenzien 24 um eine erste PCR durchzuführen. Die Reagenzien 24 lösen sich im Wasser und gelangen über eine Kanal 25, die Ventileinheit 9, den Kanal 12 in die erste PCR-Kammer 13. Hier wird eine erste, relativ unspezifische Amplifizierung von Nukleinsäure durchgeführt. Nach dem Amplifizieren in der ersten PCR-Kammer 13 wird eine Teilmenge der PCR Reaktionslösung über den Kanal 15 in einen Abmesskanal 26 der Ventileinheit 16 weiter über Kanal 17 in die Abfallkammer 18 eingeleitet. Nun wird die Ventileinheit 16 so geschaltet, dass Wasser über einen Anschluss 20 eingeleitet werden kann und über den Kanal 21 und der Ventileinheit 16 sowie den Abmesskanal 26 mit dem vorgegebenes Volumen an PCR Reaktionslösung mit den darin befindlichen amplifizierten Nukleinsäuresträngen, direkt weiter über einen Kanal 27 in eine zweite PCR-Kammer 28 geleitet wird.

In der zweiten PCR-Kammer 28 befinden sich Reagenzien 29 für die Durchführung einer zweiten PCR, in der sehr spezifisch amplifiziert wird. Die zweite PCR-Kammer 28 ist mit einem Kanal 30 verbunden, der eine Verbindung 31 nach außen umfasst, die in Form eines Ventils vorliegen kann. Durch die Verbindung 31 wird ein Druckausgleich in der zweiten PCR-Kammer 28 ermöglicht.

Die in Figur 5 gezeigte Vorrichtung umfasst einen Kanal 32, der die beiden Ventileinheiten 9 und 16 miteinander verbindet. Dieser Kanal 32 wird für die Resuspendierung der getrockneten Reagenzien in der Kammer 23 benötigt. Für das Resuspendieren fließt Wasser über den Zugang 20 und den Kanal 21 in die Ventileinheit 16 und wird dann durch den Kanal 32 in die Ventileinheit 9 geleitet. Von dort fließt das Wasser durch die Stationen 25, 23 und 22 weiter in die Ventileinheit 16 und dann weiter durch den Kanal 15 in die erste PCR Kammer 13 hinein, die so von unten befüllt wird. Eine Entlüftung der Kammer erfolgt über die Stationen 12 und 9 und den Kanal 30 über das Element 31.

Die in Figur 5 gezeigte Vorrichtung kann als Wegwerfartikel konzipiert sein, da diese aus preiswerten Materialien und klein gefertigt werden kann. Für die Durchführung des Verfahrens wird diese Vorrichtung in eine weitere Vorrichtung eingesetzt, die für eine automatisierte Durchführung des Verfahrens sorgt. Die weitere Vorrichtung betätigt und steuert dann automatisiert die Ventileinheiten (9, 16) sowie auch die Zufuhr von Puffern und Wasser.

Die Detektion der spezifischen Amplifikation in der zweiten PCR-Kammer 28 kann über Real Time Fluoreszenzdetektion oder durch hochauflösende DNA Schmelzkurvenanalyse erfolgen. Für beide Arten der Detektion wird in der zweiten PCR-Kammer 28 eine optische Vorrichtung zur Fluoreszenzdetektion integriert.

## Patentansprüche

1. Verfahren für die Vorbereitung einer biologischen Probe mit den Schritten:
- Aufschließen der biologischen Probe;
- Binden der Nukleinsäure der aufgeschlossenen Probe an magnetische Partikel, wobei die Bindung über Anionenaustauscher erfolgt;
- Unspezifisches Amplifizieren der gebundenen Nukleinsäuren, insbesondere mittels der ersten PCR einer nested-PCR;
- spezifisches Amplifizieren der aus der ersten Amplifizierung resultierenden Nukleinsäurestränge, insbesondere mit der zweiten PCR der nested-PCR.

2. Verfahren nach Anspruch 1, bei dem die gebundenen Nukleinsäuren vor dem Amplifizieren gewaschen werden.

3. Verfahren nach einem der vorhergehenden Ansprüche, bei dem Nukleinsäuren an magnetische Partikel (2) gebunden werden und vor dem Amplifizieren der Nukleinsäuren die Partikel mit den daran gebundenen Nukleinsäuren mit einem Magneten (14) magnetisch verbunden werden und der Magnet (14) mit den daran befindlichen magnetischen Partikeln (2) vor dem Amplifizieren separiert wird.

4. Verfahren nach einem der vorhergehenden Ansprüche, bei dem im Anschluss an die erste unspezifische Amplifizierung die Partikel separiert werden und nur der so erhaltene Überstand ganz oder teilweise der zweiten PCR einer nested-PCR zugeführt wird.

5. Verfahren nach einem der vorhergehenden Ansprüche, bei dem der für die Lyse eingesetzte Lysepuffer einen Salzgehalt von insgesamt weniger als 50 Millimolar aufweist.

6. Verfahren nach einem der vorhergehenden Ansprüche, bei dem der für die Lyse eingesetzte Lysepuffer einen pH-Wert von 7,5 bis 10,5 aufweist, wobei der pH-Wert vorzugsweise durch die Puffer Tris, Hepes und/ oder Mops stabilisiert ist.

7. Verfahren nach einem der vorhergehenden Ansprüche, bei dem der für die Lyse eingesetzte Lysepuffer nichtionische Detergenzien umfasst und zwar vorzugsweise 0,05 bis 0,4 Vol.-%.

8. Verfahren nach einem der vorhergehenden Ansprüche, bei dem der für die Lyse eingesetzte Lysepuffer PVP umfasst und zwar vorzugsweise bis zu 0,1 Vol.-%.

9. Verfahren nach einem der vorhergehenden Ansprüche, wobei das Verfahren in einem geschlossenen System durchgeführt wird, welches eine Lysekammer (1) sowie zwei Kammern (13, 28) umfasst, wobei in der ersten Kammer (13) die erste unspezifische Amplifizierung durchgeführt wird und in der zweiten Kammer (28) die zweite spezifische Amplifizierung durchgeführt wird.

10. Vorrichtung für die Durchführung eine Verfahrens nach einem der vorhergehenden Ansprüche mit einer Lysekammer (1) mit mit Anionenaustauschern versehenen magnetischen Partikeln (2) für das Binden von Nukleinsäuren, einer ersten PCR-Kammer (13) sowie Verbindungsmitteln (9, 10, 11, 12) für das Verbinden der Lysekammer (1) mit der ersten PCR-Kammer (13), mit einem Magneten (14) in der ersten PCR-Kammer (13) und einer zweiten PCR-Kammer (28) sowie Verbindungsmitteln für das Verbinden der ersten PCR-Kammer (13) mit der zweiten PCR- Kammer (28), mit den benötigten PCR-Reagenzien zur Durchführung einer ersten unspezifischen Amplifizierung und mit den benötigten PCR- Reagenzien (29) für die Durchführung einer zweiten spezifischen Amplifizierung, die sich in der zweiten PCR-Kammer befinden.

11. Vorrichtung nach Anspruch 10, mit Reagenzien (24), die sich in einer Reagenzienkammer (23) befinden, mit Mitteln (9, 12, 25) für das Verbinden der Reagenzienkammer (23) mit der ersten PCR-Kammer (13) sowie Mitteln (20, 21, 22) für die Zuführung von Wasser in die Reagenzienkammer.

12. Vorrichtung nach einem der vorhergehenden Vorrichtungsansprüche, die ein oder mehrere der folgenden Merkmale aufweist:
a) mit einer Abfallkammer (18), Mitteln (15, 16, 17) für das Verbinden der ersten PCR-Kammer (13) mit der Abfallkammer (18);
b) mit einer Ultraschallquelle für die Durchführung der Lyse.

## Claims

1. A method for the preparation of a biological sample comprising the steps of:
- digestion of the biological sample;
- binding the nucleic acid of the digested sample to magnetic particles, wherein binding occurs via anion exchangers;
- non-specific amplification of the bound nucleic acids, in particular via a first PCR of a nested-PCR;
- specific amplification of the nucleic acid strands resulting from the first amplification, in particular via a second PCR of a nested-PCR.

2. The method according to claim 1, wherein the bound nucleic acids are washed prior to amplification.

3. The method according to one of the preceding claims, wherein the nucleic acids are bound to magnetic particles (2) and the particles with the nucleic acids bound thereto are attached magnetically to a magnet and the magnet (14) with the magnetic particles (2) situated thereon is separated prior to the amplification.

4. The method according to one of the preceding claims, wherein the particles are separated following the first non-specific amplification and only the supernatant obtained is subjected completely or partially into the second PCR of a nested-PCR.

5. The method according to one of the preceding claims, wherein the lysis buffer used for lysis has a salt content of in total less than 50 millimolar.

6. The method according to one of the preceding claims, wherein the lysis buffer used for lysis has a pH-value of 7.5 to 10.5, wherein the pH-value is preferably stabilized by the buffers Tris, Hepes and/or Mops.

7. The method according to one of the preceding claims, wherein the lysis buffer used for lysis comprises non-ionic detergents and this preferably 0.05 to 0.4 vol.-%.

8. The method according to one of the preceding claims, wherein the lysis buffer used for lysis comprises PVP and this preferably up to 0.1 vol.-%.

9. The method according to one of the preceding claims, wherein the method is performed within a closed system, which comprises a lysis chamber (1) and two chambers (13, 28), wherein the first non-specific amplification is performed within the first chamber (13) and the second specific amplification is performed within the second chamber (28).

10. Device for performing a method according to one of the preceding claims comprising a lysis chamber (1) which comprises magnetic particles (2) furnished with anion exchangers for binding nucleic acids, a first PCR-chamber (13) as well as connection means (9, 10, 11, 12) for connecting the lysis chamber (1) with the first PCR-chamber (13), with a magnet (14) in the first PCR-chamber (13) and a second PCR-chamber (28) as well as connection means for connecting the first PCR-chamber (13) with the second PCR-chamber (28), with PCR-reagents needed to perform a first unspecific amplification and with PCR-reagents (29) needed to perform a second specific amplification, which are located in the second PCR-chamber.

11. The device according to claim 10, with reagents (24), which are located in a reagents chamber (23), with means (9, 12, 25) for connecting the reagents chamber (23) with the first PCR-chamber (13) as well as means (20, 21, 22) for supplying water to the reagents chamber.

12. The device according to one of the preceding device claims, which has one or more of the following characteristics:
a) with a waste chamber (18), means (15, 16, 17) for connecting the first PCR-chamber (13) with the waste chamber (18);
b) with an ultrasound source for performing the lysis.

## Revendications

1. Procédé de préparation d'une sonde biologique comprenant les étapes:
- d'ouverture de la sonde biologique;
- de liaison de l'acide nucléique de la sonde ouverte à des particules magnétiques, où la liaison a lieu par échangeur d'anions;
- d'amplification non spécifique des acides nucléiques liés, en particulier au moyen d'une première réaction en chaîne par polymérase (PCR) d'une PCR nichée (nested-PCR);
- d'amplification spécifique des brins d'acides nucléiques résultant de la première amplification en particulier à l'aide de la deuxième PCR de la PCR nichée.

2. Procédé selon la revendication 1, dans lequel les acides nucléiques liés sont lavés avant l'amplification.

3. Procédé selon l'une des revendications précédentes, dans lequel des acides nucléiques sont liés à des particules magnétiques (2) et avant l'amplification des acides nucléiques, les particules avec les acides nucléiques liés à celles-ci sont liées de manière magnétique à l'aide d'un aimant (14) et l'aimant (14) avec les particules magnétiques (2) situées sur celui-ci est séparé avant l'amplification.

4. Procédé selon l'une des revendications précédentes, dans lequel au terme de la première amplification non spécifique, les particules sont séparées et seul le surnageant ainsi obtenu est fourni totalement ou partiellement pour la deuxième PCR d'une PCR nichée.

5. Procédé selon l'une des revendications précédentes, dans lequel le tampon de lyse ajouté pour la lyse présente une teneur en sel totale inférieure à 50 millimoles par litre.

6. Procédé selon l'une des revendications précédentes, dans lequel le tampon de lyse ajouté pour la lyse présente une valeur de pH comprise entre 7,5 et 10,5, où la valeur de pH est de préférence stabilisée par le biais du tampon tris, hepes et/ou mops.

7. Procédé selon l'une des revendications précédentes, dans lequel le tampon de lyse ajouté pour la lyse comprend des détergents non-ioniques et de préférence de 0,05 à 0,4 % en volume.

8. Procédé selon l'une des revendications précédentes, dans lequel le tampon de lyse ajouté pour la lyse comprend de la polyvinylpyrrolidone (PVP) et de préférence jusqu'à 0,1 % en volume.

9. Procédé selon l'une des revendications précédentes, où le procédé est mis en oeuvre dans un système fermé, lequel comprend une chambre de lyse (1) ainsi que deux chambres (13, 28), où la première amplification non spécifique est effectuée dans la première chambre (13) et la deuxième amplification spécifique est effectuée dans la deuxième chambre (28).

10. Dispositif pour la mise en oeuvre d'un procédé selon l'une des revendications précédentes avec une chambre de lyse (1) avec des particules magnétiques (2) prévues avec des échangeurs d'anions pour la liaison d'acides nucléiques, une première chambre de PCR (13) ainsi que des moyens de connexion (9, 10, 11, 12) destinés à relier la chambre de lyse (1) à la première chambre de PCR (13), avec un aimant (14) dans la première chambre de PCR (13), et une deuxième chambre de PCR (28) ainsi que des moyens de connexion destinés à relier la première chambre de PCR (13) à la deuxième chambre de PCR (28), avec les réactifs de PCR appropriés pour la mise en oeuvre d'une première amplification non spécifique et avec les réactifs de PCR appropriés (29) pour la mise en oeuvre d'une deuxième amplification spécifique, qui se situent dans la deuxième chambre de PCR.

11. Préparation selon la revendication 10, avec des réactifs (24), qui se situent dans une chambre à réactifs (23), avec des moyens (9, 12, 25) destinés à relier la chambre à réactifs (23) à la première chambre de PCR (13) ainsi que des moyens (20, 21, 22) destinés à fournir de l'eau dans la chambre à réactifs.

12. Préparation selon l'une des revendications précédentes relatives à la préparation, qui présente une ou plusieurs caractéristiques parmi les suivantes:
a) avec une chambre à déchets (18), des moyens (15, 16, 17) destinés à relier la première chambre de PCR (13) à la chambre à déchets (18);
b) avec une source d'ultrasons pour la mise en oeuvre de la lyse.
